# EUROPEAN PATENT APPLICATION

(11) **EP 3 909 565 A1**
(43) Date of publication of application: **17.11.2021**
(21) Application number: 20174559.3
(22) Date of filing: 14.05.2020
(51) Int. Cl.: A61K 9/00, A61K 9/16, A61K 47/26

(54) **INHALABLE LACTOSE CONTAINING COMPOSITION**

(71) Applicant: MEGGLE GmbH & Co. KG, 83512 Wasserburg am Inn (DE)
(72) Inventor: KOBLER, Mirjam, 83026 Rosenheim (DE)
(74) Representative: Weickmann, Hans

(57) **Abstract**

The invention relates to a powder composition comprising spherically agglomerated lactose particles comprising radially arranged prism-like lactose particles, at least one active pharmaceutical ingredient (API) and optionally at least one additive, its manufacture and its use for inhalation applications.

## Description

The invention relates to a powder composition comprising spherically agglomerated lactose particles comprising radially arranged prism-like lactose particles, at least one active pharmaceutical ingredient (API) and optionally at least one additive, its manufacture and its use for inhalation applications.

The administration of APIs via the lung is becoming increasingly important due to the rising number of patients all over the world suffering from respiratory diseases. Dry powder inhalers (DPIs) are widely used in pulmonary and nasal API delivery because of their simplicity of use, small size and portability. Since formation of an API aerosol is breath-induced, DPIs are propellant-free and, thus, environmentally friendly. Furthermore, the solid-particle formulations are usually storage-stable.

Inhalable APIs for DPIs require a certain size to reach the deeper regions (alveoli) of the lung. Usually, API particles having diameters ranging from 1 to 10 µm are used. API particles with lower diameters would be breathed out again from the lung, whereas API particles having a bigger diameter would be retained in the oral cavity and would, thus, not reach the lung.

Suitable API particles can be obtained, for example, by micronization, controlled precipitation or by spray drying. However, such small particles exhibit very high intrinsic surfaces causing high cohesion forces. The cohesion forces in turn lead to high agglomeration tendencies resulting in insufficient lung deposition of API particles. Small API particles are difficult to handle due to e.g. electrostatic charge, requirements to occupational safety, limited workability and limited dosing accuracy.

It is essential to add carrier particles to powder compositions for DPI not only for dilution of the API but also to reduce cohesive forces between API particles, thereby facilitating workability and dosing accuracy. The most common carrier is lactose, but also salts, sugars, sugaralcohols or polymers can be used. Their size is to be adjusted to the respective API to be inhaled, the DPI, etc. Methods for controlling the size are known in the art and include sieving, milling, spray drying, precipitation or micronizing.

On the one hand, it is essential for a controlled drug delivery via DPI that the API is homogeneously distributed among the carrier particles, even under mechanical stress such as movement. That is, the adhesion between carrier and API must have a certain strength. The carrier particles can only adsorb a limited amount of API. At API contents above this threshold, homogeneous powder compositions cannot be prepared. Usually the powder compositions are limited to concentrations of about 0.1-5 wt.-% of API.

On the other hand, the API particles must be detached from the carrier particles during inhalation to assure the API particles to enter into the deeper regions of the respiratory tract (lung). That is, the adhesive forces between the carrier particles and the API particles are not too strong. Consequently, the adhesion is to be balanced by adjusting the physical characteristics of the API and carrier particles.

It is well known that carrier materials, such as crystalline lactose, contain defects resulting in active sites with high adhesion forces between API and carrier. Consequently, detachment of API from the carrier during the inhalation process is prevented (Kassem and Ganderton: J. Pharm. Pharmacol. 42 (1990), 11 ff.). WO 95/11666 A1 proposes to saturate these active sites by adding microfine lactose to coarse lactose carrier particles. As a result, the adhesion of the API particles to the surface is reduced.

Therefore, it became common practice to use complex systems of at least two different lactose grades and an API (so called ternary systems). However, such complex systems require significant preparation efforts and reduces flexibility.

WO 2016/039698 A1 describes particles of spherically agglomerated lactose for direct tableting compression. Said particles are said to provide high tensile strength in tablets. Pulmonary and/or nasal DPI applications are not disclosed.

Surprisingly, it has been found that spherically agglomerated lactose particles comprising radially arranged prism-like lactose particles adsorb APIs homogeneously, even at high concentrations of API. Such powder compositions result in excellent lung deposition of the API without the need of complex mixtures of carrier materials.

The object of the present invention is solved by a powder composition comprising:
(i) spherically agglomerated lactose particles comprising radially arranged prism-like lactose particles,
(ii) at least one active pharmaceutical ingredient (API), and
(iii) optionally at least one additive.

Preferably, the spherically agglomerated lactose particles are porous. They preferably have a mean average diameter of 50-800 µm, more preferably 100-300 µm.

The prism-like lactose particles preferably have a mean average diameter, which is smaller than that of the spherically agglomerated lactose particles and usually range between 0.1 to 50 µm, preferably 1 to 50 µm. Preferably, the prism-like lactose particles are crystalline. The prism-like particles are preferably in the form of prisms having a rectangular basis or trapezoidal basis. Preferably, the prism-like lactose particles are either in the form of ideal prisms or substantially ideal prisms, e.g. prisms in which the base areas are inclined to each other and/or not identical. The aspect ratio (length to width) of the prism-like lactose particle is preferably > 1, more preferably 2-10.

Preferably, the prism-like lactose particles are substantially homogenously distributed over the surface of the spherically agglomerated lactose particles. More preferably, the prism-like lactose particles are in contact with adjacent prism-like lactose particles and more preferably envelope or encapsulate the spherically agglomerated lactose particles.

Component (i) has preferably a mean average particle size from 50-1,000 µm, more preferably from 100-400 µm. The mean average particle size is measured by known methods such as sieving, light scattering and laser diffraction.

Preferably, component (i) has a dso value from 50-250 µm, more preferably from 75-200 µm. A dso value means that 50% of the particles are smaller than the specified value. Accordingly, a d₁₀ value means that 10% of the particles are smaller than the specified value and a dg0 value means that 90% of the particles are smaller than the specified value. The determination of the d₁₀/d₅₀/d₉₀ value can be carried out with methods known to the skilled person, e.g. laser diffraction, granulometry, sieving, etc. Preferably the d₁₀/d₅₀/d₉₀ values are measured according to USP (<429>) or European Pharmacopeia (2.9.31).

Preferably, component (i) has a specific surface area from 0.5-5.0 m²/g, more preferably from 0.6-3.5 m²/g. Specific surface area is defined as the total surface area of a material per unit of mass. Specific surface area can be determined by all methods known to the skilled person, for instance BET-measurements via gas adsorption according to DIN ISO 9277.

In a preferred embodiment, component (i) is obtained according to a process comprising the steps:
(a) preparing a lactose solution,
(b) adding the lactose solution obtained after step (a) to at least one non-solvent under constant stirring to form spherically agglomerated lactose particles comprising radially arranged prism-like lactose particles,
(c) isolating the spherically agglomerated lactose particles, and
(d) optionally drying.

Preferably, the lactose solution is an aqueous solution.

Preferably, lactose is dissolved in the solvent in step (a) resulting in a concentration of 0.1-75 wt.-%, more preferably 7.5-55.0 wt.-%, more preferably 17.5-35.0 wt.-%. The temperature of the lactose solution is preferably in the range from -10-90 °C, more preferably 25-65 °C, more preferably 35-50 °C.

The non-solvent in step (b) is preferably selected from the group consisting of methanol, ethanol, n-propanol, 2-propanol and n-butanol, more preferably ethanol and 2-propanol, more preferably ethanol. The non-solvent may comprise up to 50 wt.-%, preferably 1-30 wt.-% of water. Preferably the temperature of the non-solvent is in the range from -20-115 °C, more preferably 25-65 °C, more preferably 35-50 °C. Preferably, the lactose solution is added to an excess (v/v) of non-solvent or aqueous non-solvent.

Preferably, adding the lactose solution obtained after step (a) to at least one non-solvent in step (b) is performed at a flow rate ranging from 1-10 mL/min, more preferably 2-8 mL/min, more preferably 4-6 mL/min. Preferably, adding the lactose solution obtained after step (a) to at least one non-solvent in step (b) is performed under stirring.

The mixture obtained after step (b) may preferably be stirred for 10-90 min, more preferably 15-60 min, more preferably 20-40 min before step (c).

Isolating the spherically agglomerated lactose particles in step (c) is preferably performed by centrifugation or filtration, more preferably filtration.

Drying in step (d) is - if at all - preferably carried out at a temperature from 20 to 130 °C, more preferably 25-100 °C, more preferably 30-70 °C. Drying in step (d) is preferably carried out for 3-36 h, more preferably 4-24 h, more preferably 5-12 h. Drying in step (d) may be performed with all drying means known to the skilled person, preferably using vacuum drying, a laboratory dryer, rotary dryer or fluid bed dryer.

Spherically agglomerated lactose particles comprising radially arranged prism-like lactose particles are most preferably those described in WO2016/039698, which is herein incorporated by reference.

Lactose is a disaccharide consisting of the monosaccharides galactose and glucose. Lactose can occur in two different isomeric forms, namely α-lactose and β-lactose. α-lactose (formula 1) differs from β-lactose (formula 2) in the position of the hydroxy group on the C₁ atom of the glucose molecule.

In a preferred embodiment, component (i) comprises α-lactose, β-lactose or combinations thereof, preferably α-lactose.

Lactose may be present as anhydrous lactose, lactose monohydrate or combinations thereof, preferably lactose monohydrate.

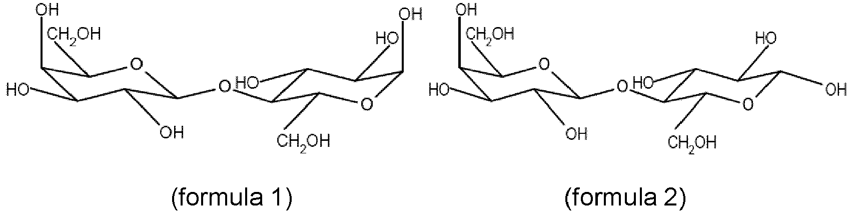

According to the invention, lactose of a certain isomeric form can still contain up to 5 wt.-%, preferably up to 3 wt.-%, of amorphous lactose or lactose of the other isomeric form.

The API in component (ii) is preferably selected from a respiratory and systemic active pharmaceutical ingredient, more preferably for the treatment of diseases of the respiratory system, diseases caused by bacteria, diseases of the cardiovascular system, allergic reactions, cancer, psychiatric diseases and immune system related diseases. A respiratory API is an API being locally effective in the respiratory tract. A systemic API is an API, which distributes within the whole organism via the blood- and/or lymphatic system after administration.

The active pharmaceutical ingredient for the treatment of diseases of the respiratory system is preferably selected from the group consisting of inhaled corticosteorides (ICS), short acting beta agonists (SABA), long acting beta agonists (LABA), short acting anticholinergic (SAMA), long acting anticholinergic (LAMA), bronchodilators and antitussives, more preferably salbutamol, budenoside, formoterol, salmeterol, ipatropiumbromid, terbutaline, tiotropium, indaceterol, umcledinium, glycopyrronium, aclidinium, beclometasone, fluticasone, vilanterol, and pharmaceutically acceptable salts thereof.

The active pharmaceutical ingredient for the treatment of diseases caused by bacteria is preferably selected from the group consisting of β-lactams, fluorquinolones, macrolides, tetracyclines, aminoglycosides, lincosamides, oxazolidinones and glycopeptides, more preferably rifampicin, amoxiclin, moxifloxacin, levofloxacin, clarithromycin, doxycyclin, amipicillin, cefuroxim, ceftriaxon, cefotaxim, etrapenem, metropenem, imipenem, ciprofloxacin, gentamicin, tobraycin, amikacin, ceftobiprol, ceftazidium, flucloaxacillin, penicillin, cefazolin, clindamycin, linezolid, vacomiycin and pharmaceutically acceptable salts thereof.

The active pharmaceutical ingredient for the treatment of diseases of the cardiovascular system is preferably selected from the group consisting of ACE inhibitors, aldosterone receptor antagonists, beta blockers, cardiac glycosides, diuretics, neprilysin inhibitors and organic nitro compounds, more preferably benazapril, captopril, cilazapril, enalapril, ramipril, spironolactone, canrenone, eplerenone, propranolol, bucindolol, acebutolol, bisoprolol, butaxamine, nebivolol, amiloride, triamterene, nitroglycerine and pharmaceutically acceptable salts thereof.

The active pharmaceutical ingredient for the treatment of allergic reactions is preferably selected from the group consisting of antihistamines and mast cell stabilizers, more preferably acrivastine, astemizole, cetirizine, ebastine, loratadine, mizolastine, terfenadine, rupatadine, cromoglicic acid, nedocromil and pharmaceutically acceptable salts thereof.

The active pharmaceutical ingredient for the treatment of cancer is preferably selected from the group consisting of alkylating antineoplastic agents, platinum analogs, anthracyclines, mitosis inhibitors, taxanes, topoisomerase inhibitors, kinase inhibitors, cellular-based ingredients, mRNA-based ingredients, DNA-based ingredients, vector-based ingredients, polyplex-based ingredients, protein-based ingredients, monoclonal antibodies and polyclonal antibodies, more preferably cyclophosphamide, carboplatin, cisplatin, idarubicin, daunorubicin, doxorubicin, mitoxantron, epirubicin, pixantron, paclitaxel, docetaxel, cabazitaxel and pharmaceutically acceptable salts thereof.

The active pharmaceutical ingredient for the treatment of psychiatric diseases is preferably selected from the group consisting of antidepressants, mood stabilizers, neuroleptics and anxiolytics, more preferably agomelatine, paroxetine, citalopram, amitriptyline, aripiprazole, clozapine, olanzapine, quetiapine, risperidone and pharmaceutically acceptable salts thereof.

The active pharmaceutical ingredient for the treatment of immune system related diseases is preferably an immune suppressive API, more preferably selected from the group consisting of glucocorticoid, cytostatic agent, antibody and API acting on immunophilins.

Preferably, the API of component (ii) is particulate.

Preferably, component (ii) has a particle size distribution dso as described above of 1-10 µm, preferably 1-5 µm.

API, in particular API particles, of component (ii) is preferably adsorbed on component (i), more preferably on the surface of component (i), more preferably on the surface of the prism-like lactose particles. More preferably, API particles of component (i) are essentially homogeneously distributed over the surface of component (i).

The additive in component (iii) preferably acts as force controlling agent (FCA) and is preferably selected from a lubricant and a stabilizing agent. FCAs represent possible means of decreasing interparticulate interactions of API particles.

A lubricant is a substance introduced to reduce friction between surfaces in mutual contact. Preferably, the lubricant is magnesium stearate.

A stabilizing agent is used to prevent degradation of the powder composition, in particular due to microbial, radiation-induced and humidity-induced degradation. Preferably, the stabilizing agent is a carbohydrate, amino acid or metal salt, more preferably lactose, mannitol, trehalose or leucine.

Preferably, the overall powder composition has a dso value in the range from 50-1,000 µm, more preferably from 50-300 µm, more preferably from 50-150 µm.

Fine particle dose (FPD) and fine particle fraction (FPF), respectively, are performance characteristics for DPI systems. FPD is the mass of API particles < 5 µm in diameter, preferably < 3.5 µm in diameter within the total emitted API dose during an inhaling event (shot) and, thus, represents the respirable dose. FPF is calculated from the FPD divided by the total mass of the emitted API dose (dose per shot).

FPD/FPF can be determined by Impaction, according to compendial methods as described in general chapters of USP (<601>) and Ph. Eur. (2.9.18). For instance, a Next Generation Pharmaceutical Impactor (NGI) is used.

Preferably, the overall powder composition has a FPF ranging from 20-90%, more preferably from 30-80%, more preferably from 40-70%.

Preferably, the overall powder composition has a mass median aerodynamic diameter (MMAD) ranging from 0.5-5 µm, more preferably from 1-4 µm, more preferably from 1.5-3 µm.

The aerodynamic diameter of a certain particle is defined as the diameter of a spherical particle with a density of 1 g/cm³, which has the same sink rate as the investigated particle. The mass median aerodynamic diameter (MMAD) represents the aerodynamic diameter for which 50% of the total mass of all particles in a given aerosol are particles smaller than the MMAD. The MMAD plays an important role in inhalation therapy. While particles with an MMAD > 10 µm are already deposited at the throat due to their inertia, particles having a MMAD < 1 µm are breathed out again from the lung. Particles having MMADs between 1-10 µm optimally sediment/deposit in the respiratory system reaching the deeper regions of the lung (alveoli).

The amount of component (i) in the powder composition is preferably in the range of 50-99.9 wt.-%, more preferably in the range of 80-99.9 wt.-%, based on the total amount of components (i) and (ii).
The amount of component (ii) in the powder composition is preferably in the range from 0.1-50 wt.-%, more preferably in the range from 0.1-20 wt.-%, based on the total amount of components (i) and (ii).

Preferably, component (iii) in the powder composition is present in the range from 0.1-20 wt.-%, more preferably in the range from 0.1-10 wt.-%, based on the total amount of components (i), (ii) and (iii).

Preferably, the powder composition is not in form of a tablet or injective, more preferably not in form of a tablet.

Another aspect of the present invention relates to a method of preparing the above-described powder composition, comprising the steps of:
(1) adding components (ii) and optionally (iii) to component (i),
(2) mixing the product obtained after step (1), and
(3) optionally sieving the mixture obtained after step (2).

Preferably, components (i), (ii) and/or optionally (iii) are sieved prior to step (1). More preferably, components (i), (ii) and/or optionally (iii) are sieved with a sieve having a mesh size in the range from 30-500 µm, more preferably from 150-400 µm.

Step (2) is preferably performed in a stirrer, high shear mixer, low shear mixer and/or by co-processing.

Preferably, a sieve having a mesh size in the range from 30-500 µm, more preferably from 150-400 µm is used in step (3).

Another aspect of the present invention relates to the powder composition as described above for use as inhalable medicament and/or inhalable diagnostic agent.

Another aspect of the present invention relates to the powder composition as described above for use in the treatment of diseases of the respiratory system, diseases caused by bacteria, diseases of the cardiovascular system, allergic reactions, cancer, psychiatric diseases and/or immune system related diseases. Another aspect of the present invention relates to the use of component (i) as carrier in inhaler compositions.
- **Figure 1**: SEM images of powder composition (III) at a magnification of 250 (A), 500 (B), 1000 (C), 2500 (D) and 5000 (E).
- **Figure 2**: SEM images of powder composition (IV) at a magnification of 250 (A), 500 (B), 1000 (C), 2500 (D) and 5000 (E).
- **Figure 3**: SEM images of powder composition (V) at a magnification of 250 (A), 500 (B), 1000 (C), 2500 (D) and 5000 (E).
- **Figure 4**: SEM images of powder composition (VI) at a magnification of 250 (A), 500 (B), 1000 (C), 2500 (D) and 5000 (E).
- **Figure 5**: SEM images of powder composition (VII) at a magnification of 500 (A), 1000 (B), 2500 (C) and 5000 (D).
- **Figure 6**: SEM pictures of component (i) used in Example 8.
- **Figure 7**: Particle size distribution of component (i) used to prepare powder composition (VIII).
- **Figure 8**: d₅₀ values (A), span (B), percentage of particles < 5 µm (C) and specific surface area (D) of component (i) (=SL) compared to commercially available lactose carriers INH70, INH120, INH230 and INH250.
- **Figure 9**: FPF values determined for powder compositions comprising component (i) (=SL) compared to comparative powder compositions A-D (cf. example 8). All powder compositions further comprise salbutamol sulfate (SBS).
The present invention is illustrated by the following examples, without limitation thereto.

### Example 1: Powder composition (I)

Powder composition (I) consists of lactose agglomerates according to component (i) of the invention (prepared according to Example 7 in WO 2016/039698) and 1.5 wt.-% of salbutamol sulfate as API according to component (ii) of the invention.

### Preparation of powder composition (I)

Salbutamol sulfate was sieved with a 180 µm sieve to remove agglomerates. 0.45 g sieved salbutamol sulfate was added to 29.55 g component (i) (total batch mass of 30 g) followed by mixing with an Alpine Picoline equipped with the Picomix® high shear mixer module (Hosokawa Alpine, Augsburg, Germany) at 500 rpm for one minute. After mixing, the product was passed through a 355 µm sieve to receive powder composition (I).

### Blend homogeneity and API recovery

Homogeneity was assessed by measuring the API content of ten randomly drawn aliquots of powder composition (I) by an HPLC method.

Details regarding the HPLC approach are given below:

| | |
|---|---|
| Analyte: | Salbutamol sulfate / dissolved in water |
| Instrumentation: | Agilent 1100 Series HPLC Value System |
| Mobile phase: | 78 wt.-% buffering system containing 2.87 g/L sodium heptanesulfonate and 2.5 g/L KH₂PO₄ (0.2mmol), pH adjusted to 3.65 with ortho-phosphoric acid 85 wt.-% 22 wt.-% acetonitrile |
| Flow: | 0.8 mL/min |
| Column: | LichroCART 125-4, Lichrospher RP-18 (5µm) with precolumn |
| Wavelength: | 220 nm |
| Retention time: | ∼3 min |
| Calibration: | 1-100 µg/mL |
| Injection Volume: | 100 µL |
| Oven temperature: | 25°C |

HPLC experiments resulted in an API recovery of 98.11 ± 2.88% over all investigated aliquots and thus confirmed excellent blend homogeneity.

### Aerodynamic assessment of the powder composition by a Next Generation Pharmaceutical Impactor (NGI) test.

Powder composition (I) was stored at standard conditions (1 bar, 20°C) for a minimum of four weeks before it was tested for aerodynamic performance with a Novolizer® coupled to NGI instrumentation (Marple et al., Journal of Aerosol Medicine, 2003, 16, 283-299) forming the "device".

NGI is a cascade impactor, which has been specifically designed for pharmaceutical inhaler testing. The NGI has seven stages and is intended to assess aerodynamic properties of powder compositions. These properties follow established scientific principles, giving reliable particle size fractionation.

By applying a pressure drop over the device and providing the powder composition to the inlet of the NGI, inhaling the powder composition by a patient can be simulated.

The airflow required to create a 4 kPa pressure drop over the device was obtained via a vacuum pump at an air flow rate of 78.3 L/min.

After simulating an inhalation event, API-particles are distributed among the stages of the NGI and deposited on collection cups that are held in a tray. This tray is removed from the NGI as a single unit. Solvent is added to each single cup corresponding to a certain stage in the NGI for dissolving the deposited API. HPLC quantification of the solutions derived from each stage is used for identifying API distribution within the stages.

The amount of API per inhaled dose was determined as follows. The Novolizer® was filled with powder composition (I) and weighed using an analytical balance (AT106 Comparator, Mettler Toledo, Switzerland). Afterwards the Novolizer® was fitted into a customized mouthpiece, connected to the NGI, actuated (flow rate for pressure drop of 4 kPa, 4 L inspiration volume) into the NGI and weighed again. The delivered mass of ten single shots was calculated from the difference of the weight before and after every actuation.

The following table lists the shot weight of each shot number. Reproducible shot weights become apparent.

**Table1: Shot weight test; powder composition (I).**

| Shot number | Shot weight |
|---|---|
| 1 | 11.20 mg |
| 2 | 10.71 mg |
| 3 | 10.49 mg |
| 4 | 10.81 mg |
| 5 | 10.88 mg |
| 6 | 10.57 mg |
| 7 | 10.71 mg |
| 8 | 10.86 mg |
| 9 | 10.62 mg |
| 10 | 10.45 mg |
| **average:** | 10.73 mg |
| **abs. standard deviation:** | 0.22 mg |
| **rel. standard deviation:** | 2.06 % |

Aerodynamic particle size distribution was determined by cascade impaction. The NGI equipped with a trigger box and a vacuum pump HCP 5 (all Copley Scientific, UK) was used. The air flow was set to a flow rate resulting in a pressure drop of 4 kPa across the device (depending on the device resistance). Afterwards the airflow was applied over a corresponding time ensuring 4 L of air to be withdrawn from the mouthpiece of the inhaler. A stage coating composed of Brij®35, glycerol, and absolute ethanol was applied onto each stage of the impactor before analysis to reduce bouncing phenomena.

After connecting the Novolizer® to the induction port of the NGI via a matching adapter, 5 shots were delivered to the NGI to allow sufficient sample for API quantification.

Novolizer®, induction port, preseparator and all stages were individually sampled for API. A defined volume of solvent was used for each sample.

| | |
|---|---|
| Novolizer® | 10 mL |
| Induction port | 15 mL |
| Preseparator | 20 mL |
| Stages 1-7 | 5 mL |
| MOC | 5mL |

Sufficient dwell times were applied to ensure complete API dissolution. All samples were not further diluted and immediately subjected to HPLC analysis. The calculation of the fine particle dose (FPD), fine particle fraction (FPF) and mass median aerodynamic diameter (MMAD) was performed with the CITDAS 3.0 software (Copley Scientific, UK). The calculated fine particle fraction (FPF) is expressed as the FPF of the delivered dose, i.e. the percentage of total amount delivered to the NGI while the retention of API sticking to the Novolizer® is not considered.

Table 2 and 3 summarize the outcome of the NGI testing, namely the API mass per stage of each single run (Table 2) and as average data (Table 3).

**Table 2: API mass per stage; mass distribution; single run data (5 shots).**

| **Stage** | **Stage deposition, µg** | | |
|---|---|---|---|
| | Run 1 | Run 2 | Run 3 |
| **Novolizer®** | 36.57 | 32.24 | 25.24 |
| **Induction port** | 146.29 | 121.60 | 127.79 |
| **Preseparator** | 153.82 | 177.90 | 175.06 |
| **Stage 1** | 18.28 | 23.96 | 19.26 |
| **Stage 2** | 30.86 | 33.72 | 30.84 |
| **Stage 3** | 73.45 | 73.50 | 70.95 |
| **Stage 4** | 120.63 | 126.32 | 127.23 |
| **Stage 5** | 87.98 | 91.37 | 97.86 |
| **Stage 6** | 42.57 | 44.12 | 43.43 |
| **Stage 7** | 16.96 | 12.82 | 15.77 |
| **MOC** | 8.50 | 3.38 | 5.26 |

**Table 3: API mass per stage; average data (3 runs).**

| **Stage** | **Stage deposition, average, µg** | **Stage deposition, absolute standard deviation, µg** | **Stage deposition, relative standard deviation, %** |
|---|---|---|---|
| **Novolizer®** | 31.35 | 5.71 | 18.23 |
| **Induction Port** | 131.89 | 12.85 | 9.74 |
| **Preseparator** | 168.93 | 13.16 | 7.79 |
| **Stage 1** | 20.50 | 3.04 | 14.82 |
| **Stage 2** | 31.81 | 1.66 | 5.21 |
| **Stage 3** | 72.63 | 1.46 | 2.01 |
| **Stage 4** | 124.72 | 3.58 | 2.87 |
| **Stage 5** | 90.41 | 2.11 | 2.34 |
| **Stage 6** | 43.37 | 0.77 | 1.78 |
| **Stage 7** | 15.18 | 2.13 | 14.04 |
| **MOC** | 5.71 | 2.59 | 45.32 |

Tables 4 and 5 present above-given results in a relative manner based on the total mass of API entering the NGI.

**Table 4: Relative stage distribution; single run data.**

| | **Stage deposition, %** | | |
|---|---|---|---|
| | Run 1 | Run 2 | Run 3 |
| **Novolizer®** | 4.97 | 4.35 | 3.45 |
| **Induction port** | 19.88 | 16.41 | 17.44 |
| **Preseparator** | 20.90 | 24.01 | 23.89 |
| **Stage 1** | 2.48 | 3.23 | 2.63 |
| **Stage 2** | 4.19 | 4.55 | 4.21 |
| **Stage 3** | 9.98 | 9.92 | 9.68 |
| **Stage 4** | 16.39 | 17.05 | 17.36 |
| **Stage 5** | 11.96 | 12.33 | 12.54 |
| **Stage 6** | 5.79 | 5.95 | 5.93 |
| **Stage 7** | 2.30 | 1.73 | 2.15 |
| **MOC** | 1.16 | 0.46 | 0.72 |

**Table 5: Relative stage distribution: average data (3 runs).**

| **Stage** | **Stage deposition, average, %** | **Stage deposition, absolute standard deviation, %** | **Stage deposition, relative standard deviation, %** |
|---|---|---|---|
| **Novolizer®** | 4.26 | 0.77 | 18.01 |
| **Induction Port** | 17.91 | 1.78 | 9.94 |
| **Preseparator** | 22.93 | 1.76 | 7.68 |
| **Stage 1** | 2.78 | 0.40 | 14.30 |
| **Stage 2** | 4.32 | 0.20 | 4.68 |
| **Stage 3** | 9.86 | 0.16 | 1.59 |
| **Stage 4** | 16.93 | 0.50 | 2.93 |
| **Stage 5** | 12.28 | 0.30 | 2.41 |
| **Stage 6** | 5.89 | 0.09 | 1.55 |
| **Stage 7** | 2.06 | 0.30 | 14.43 |
| **MOC** | 0.78 | 0.35 | 45.47 |

Determined aerodynamic parameters are presented in Tables 6 and 7.

**Table 6: Aerodynamic parameters, single shot data (calculated).**

| | **Run 1** | **Run 2** | **Run 3** |
|---|---|---|---|
| **Recovered dose per shot, ug** | 147.18 | 148.19 | 146.54 |
| **Calc. delivered dose per shot, µg** | 139.87 | 141.74 | 141.49 |
| **Fine particle dose per shot, µg** | 73.22 | 73.72 | 74.08 |
| **Fine particle fraction, %** | **52.35** | **52.01** | **52.36** |
| **MMAD, µm** | 1.77 | 1.82 | 1.77 |

**Table 7: Aerodynamic parameters, average data (from 3 runs).**

| | **average** | **absolute standard deviation** | **relative standard deviation, %** |
|---|---|---|---|
| **Recovered dose per shot, µg** | 147.30 | 0.83 | 0.56 |
| **Calc. delivered dose per shot, µg** | 141.03 | 1.02 | 0.72 |
| **Fine particle dose per shot, µg** | 73.67 | 0.43 | 0.59 |
| **Fine particle fraction, %** | **52.24** | **0.20** | **0.38** |
| **MMAD, µm** | 1.78 | 0.03 | 1.82 |

Powder composition (I) showed a homogeneous distribution of the API. Further, owing to very low variations in the shot weights, good dosing capability of powder composition (I) with the Novolizer® can be achieved. Moreover, as high FPF and good API distribution during the NGI testing could be observed, excellent aerodynamic properties and lung deposition of powder composition (I) becomes apparent. The small MMAD ensures optimal deposition of the API in deeper regions of the lung (alveoli).

### Example 2: Powder composition (II)

Powder composition (II) consists of lactose agglomerates according to component (i) of the invention (prepared according to Example 7 in WO 2016/039698) and 1.5 wt.-% of budenoside as API according to component (ii) of the invention.

### Preparation of powder composition (II)

Powder composition (II) was prepared corresponding to powder composition (I), except that budenoside was used as API instead of salbutamol sulfate.

### Blend homogeneity and API recovery

Homogeneity was assessed similar to powder composition (I).

Details regarding the HPLC approach are given below:

| | |
|---|---|
| Analyte: | budenoside / dissolved in methanol:water (75:25) |
| Instrumentation: | Agilent 1100 Series HPLC Value System |
| Mobile phase: | 75 wt.-% methanol |
| | 25 wt.-% water |
| Flow: | 1.0 mL/min |
| Column: | LiChrospher RP select B (5 µm) LiChroCART125-4 with precolumn |
| Wavelength: | 248 nm |
| Retention time: | ∼4 min |
| Calibration: | 1-100 µg/mL |
| Injection Volume: | 100 µL |
| Oven temperature: | 25°C |

HPLC experiments resulted in an API recovery of 99.01 ± 2.88% over all investigated aliquots and thus confirmed excellent blend homogeneity.

### Aerodynamic assessment of the powder composition by a Next Generation Pharmaceutical Impactor (NGI) test.

Aerodynamic assessment of powder composition (II) was performed as described for powder composition (I) above.

The following table lists the shot weight of each shot number. Reproducible shot weights become apparent.

**Table 8: Shot weight test; powder composition (I).**

| Shot number | Shot weight |
|---|---|
| 1 | 11.20 mg |
| 2 | 10.71 mg |
| 3 | 10.49 mg |
| 4 | 10.81 mg |
| 5 | 10.88 mg |
| 6 | 10.57 mg |
| 7 | 10.71 mg |
| 8 | 10.86 mg |
| 9 | 10.62 mg |
| 10 | 10.45 mg |
| **average:** | 10.73 mg |
| **abs. standard deviation:** | 0.22 mg |
| **rel. standard deviation:** | 2.06 % |

Tables 9 and 10 summarize the outcome of the NGI testing, namely the API mass per stage of each single run (Table 10) and as average data (Table 11).

**Table 9: API mass per stage; mass distribution; single run data (5 shots).**

| **Stage** | **Stage deposition, µg** | | |
|---|---|---|---|
| | Run 1 | Run 2 | Run 3 |
| **Novolizer®** | 43.84 | 49.74 | 44.82 |
| **Induction port** | 135.99 | 149.67 | 153.46 |
| **Preseparator** | 188.87 | 181.80 | 168.03 |
| **Stage 1** | 41.84 | 39.94 | 28.51 |
| **Stage 2** | 69.61 | 68.36 | 67.44 |
| **Stage 3** | 86.70 | 93.90 | 89.29 |
| **Stage 4** | 86.02 | 82.83 | 87.41 |
| **Stage 5** | 48.43 | 48.36 | 53.20 |
| **Stage 6** | 24.44 | 22.88 | 23.20 |
| **Stage 7** | 6.68 | 5.28 | 7.46 |
| **MOC** | 1.41 | 1.02 | 1.57 |

**Table 10: API mass per stage; average data (3 runs).**

| **Stage** | **Stage deposition, average, µg** | **Stage deposition, absolute standard deviation, µg** | **Stage deposition, relative standard deviation, %** |
|---|---|---|---|
| **Novolizer®** | 46.14 | 3.16 | 6.85 |
| **Induction Port** | 146.37 | 9.19 | 6.28 |
| **Preseparator** | 179.57 | 10.60 | 5.90 |
| **Stage 1** | 36.76 | 7.21 | 19.61 |
| **Stage 2** | 68.47 | 1.09 | 1.59 |
| **Stage 3** | 89.96 | 3.65 | 4.06 |
| **Stage 4** | 85.42 | 2.35 | 2.75 |
| **Stage 5** | 49.99 | 2.77 | 5.55 |
| **Stage 6** | 23.51 | 0.83 | 3.51 |
| **Stage 7** | 6.47 | 1.11 | 17.08 |
| **MOC** | 1.33 | 0.28 | 21.15 |

Tables 11 and 12 present above-given results in a relative manner based on the total mass of API entering the NGI.

**Table 11: Relative stage distribution; single run data.**

| | **Stage deposition, %** | | |
|---|---|---|---|
| | Run 1 | Run 2 | Run 3 |
| **Novolizer®** | 5.97 | 6.69 | 6.19 |
| **Induction Port** | 18.53 | 20.12 | 21.18 |
| **Preseparator** | 25.74 | 24.44 | 23.20 |
| **Stage 1** | 5.70 | 5.37 | 3.94 |
| **Stage 2** | 9.49 | 9.19 | 9.31 |
| **Stage 3** | 11.81 | 12.63 | 12.33 |
| **Stage 4** | 11.72 | 11.14 | 12.07 |
| **Stage 5** | 6.60 | 6.50 | 7.34 |
| **Stage 6** | 3.33 | 3.08 | 3.20 |
| **Stage 7** | 0.91 | 0.71 | 1.03 |
| **MOC** | 0.19 | 0.14 | 0.22 |

**Table 12: Relative stage distribution; average data (3 runs).**

| **Stage** | **Stage deposition, average, %** | ***Stage deposition, absolute standard deviation, %** | **Stage deposition, relative standard deviation, %** |
|---|---|---|---|
| **Novolizer®** | 6.28 | 0.37 | 5.82 |
| **Induction Port** | 19.95 | 1.34 | 6.70 |
| **Preseparator** | 24.46 | 1.27 | 5.20 |
| **Stage 1** | 5.00 | 0.94 | 18.76 |
| **Stage 2** | 9.33 | 0.15 | 1.59 |
| **Stage 3** | 12.26 | 0.41 | 3.34 |
| **Stage 4** | 11.64 | 0.47 | 4.04 |
| **Stage 5** | 6.81 | 0.46 | 6.76 |
| **Stage 6** | 3.20 | 0.13 | 3.98 |
| **Stage 7** | 0.88 | 0.16 | 18.31 |
| **MOC** | 0.18 | 0.04 | 22.33 |

Determined aerodynamic parameters are presented in Tables 13 and 14.

**Table 13: Aerodynamic parameters, single shot data (calculated).**

| | **Run 1** | **Run 2** | **Run 3** |
|---|---|---|---|
| **Recovered dose per shot, µg** | 146.77 | 148.76 | 144.88 |
| **Calc. delivered dose per shot, µg** | 138.00 | 138.81 | 135.91 |
| **Fine particle dose per shot, µg** | 57.68 | 57.70 | 59.48 |
| **Fine particle fraction, %** | **41.79** | **41.57** | **43.76** |
| **MMAD, µm** | 2.68 | 2.73 | 2.55 |

**Table 14: Aerodynamic parameters, average data (from 3 runs).**

| | **average** | **absolute standard deviation** | **relative standard deviation, %** |
|---|---|---|---|
| **Recovered dose per shot, µg** | 146.80 | 1.94 | 1.32 |
| **Calc. delivered dose per shot, µg** | 137.91 | 1.49 | 1.09 |
| **Fine particle dose per shot, µg** | 58.28 | 1.03 | 1.77 |
| **Fine particle fraction, %** | **42.37** | **1.21** | **2.85** |
| **MMAD, µm** | 2.65 | 0.09 | 3.48 |

Powder composition (II) showed a homogeneous distribution of the API. Further, owing to very low variations in the shot weights, good dosing capability of powder composition (II) with the Novolizer® can be achieved. Moreover, as high FPF and good API distribution during the NGI testing could be observed, excellent aerodynamic properties and lung deposition of powder composition (II) becomes apparent. The small MMAD ensure optimal API deposition in deeper regions of the lung (alveoli).

### Example 3. 4: Powder compositions (III) and (IV)

Powder composition (III) and (IV), respectively, consist of lactose agglomerates according to component (i) of the invention (prepared according to Example 7 in WO 2016/039698) and 1.5 wt.-% salbutamol sulfate and 10 wt.-% salbutamol sulfate, respectively, as API according to component (ii) of the invention. In contrast to powder compositions (I) and (II), a low shear mixing method with a Turbula mixer instead of a high shear mixing method was applied.

### Preparation of powder compositions (III) and (IV)

Salbutamol sulfate was sieved with a 180 µm sieve and component (i) with a 355 µm sieve to remove agglomerates. Afterwards, salbutamol sulfate and component (i) were weighed into a stainless steel cylinder in different amounts to obtain a salbutamol sulfate content of 1.5 wt.-% and 10 wt.-% , respectively, for a batch size of 30.0 g in total. Each blend was subjected to low shear mixing using the Turbula-Blender (model T2C, Willy Bachofen AG Maschinenfabrik, Basel, Switzerland) three times for each 5 minutes at 90 U/min. Between each mixing step and at the end of the mixing, the powders were sieved with a 355 µm sieve to eventually receive powder compositions (III) and (IV).

### Blend homogeneity and API recovery

Homogeneity was assessed by measuring the API content of ten randomly drawn aliquots of powder composition (III) and (IV), respectively, by an HPLC method.

Details regarding the HPLC approach are given below:

| | |
|---|---|
| Analyte: | Salbutamol sulfate / dissolved in a mixture of 78% buffer solution containing 2.87 g/L sodium heptanesulfonate and 2.50 g/L KH₂PO₄ (0.2 mmol). The pH was adjusted to 3.65 with orthophosphoric acid 85% and 22% acetonitrile. |
| Instrumentation: | Agilent 1100 Series HPLC Value System |
| Mobile phase: | 78 wt.-% buffering system containing 2.87 g/L sodium heptanesulfonate and 2.5 g/L KH2PO4 (0.2mmol), pH adjusted to 3.65 with ortho-phosphoric acid 85 wt.-% 22 wt.-% acetonitrile |
| Flow: | 1 mL/min |
| Column: | Column LiChrospher® 100 RP-18 (5 µm) (Merck, Germany) |
| Wavelength: | 220 nm |
| Retention time: | ∼2.5 min |
| Calibration: | 0.6-150 µg/mL (external standards and samples were injected twice into the HPLC) |
| Injection Volume: | 100 µL |
| Oven temperature: | 25°C |

HPLC experiments for both powder composition (III) and (IV) resulted in an API recovery of 98.98 ± 1.89% and 99.97 ± 2.89%, respectively over all investigated aliquots and, thus, confirmed excellent blend homogeneity.

### Scanning electron microscopy

Scanning electron microscopy (SEM) was carried out with a Phenom XL (Phenom-World BV, Netherlands). Particles were fixed on carbon stickers and sputter-coated with a thin gold layer prior to SEM evaluation (5 kV).

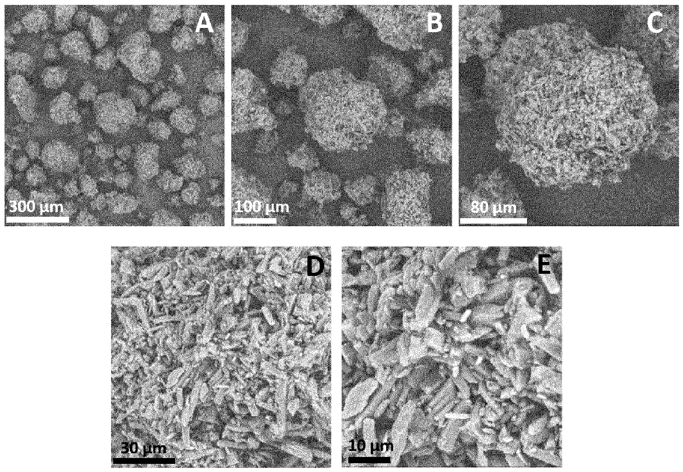

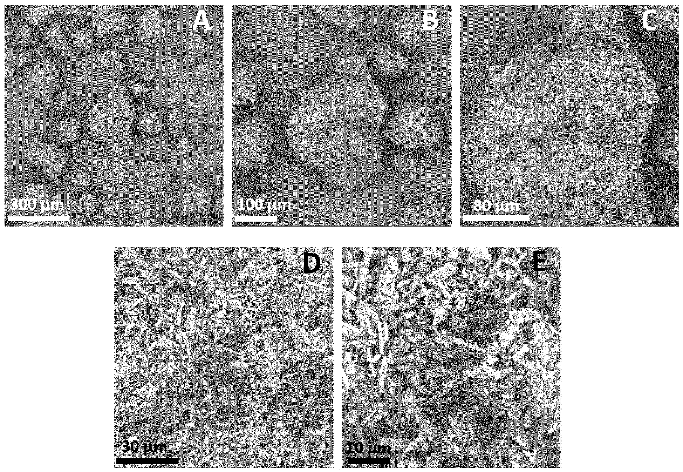

Figure 1 depicts SEM pictures of powder composition (III). Figure 2 depicts SEM pictures of powder composition (IV). For both compositions, spherically agglomerated lactose particles comprising radially arranged prism-like lactose particles carrying API particles thereon become apparent.

### Aerodynamic assessment by NGI

Powder compositions (III) and (IV) were tested for aerodynamic performance with a Novolizer®. Each formulation was stored at standard conditions for a minimum of four weeks before aerodynamic assessment.

### Shot weight test

The amount of API per inhaled dose was determined as presented in Example 1. The following tables list the shot weight of each shot number. Reproducible shot weights become apparent for both powder composition (III) and (IV).

**Table 15: API content and relative standard deviation of 10 random aliquots of powder composition (III).**

| | Sample weight [mg] | Expected API [mg] | Found API [mg] | Recovery [%] |
|---|---|---|---|---|
| | 9.98 | 0.15 | 0.15 | 103.03 |
| | 10.08 | 0.15 | 0.15 | 98.22 |
| | 10.03 | 0.15 | 0.15 | 96.92 |
| | 10.04 | 0.15 | 0.15 | 100.43 |
| | 10.20 | 0.15 | 0.15 | 98.22 |
| | 9.86 | 0.15 | 0.15 | 99.30 |
| | 10.44 | 0.16 | 0.15 | 98.52 |
| | 10.05 | 0.15 | 0.15 | 98.71 |
| | 10.33 | 0.15 | 0.15 | 97.86 |
| | 10.23 | 0.15 | 0.15 | 98.61 |
| **Mean [mg]** | 10.12 | 0.15 | 0.15 | 98.98 |
| Standard deviation [mg] | 0.17 | 0.00 | 0.00 | |
| Relative standard deviation [%] | | | 1.89 | |

**Table 16: API content and relative standard deviation of 10 random aliquots of powder composition (IV).**

| | Sample weight [mg] | Expected API [mg] | Found API [mg] | Recovery [%] |
|---|---|---|---|---|
| | 10.31 | 1.03 | 1.00 | 96.59 |
| | 10.23 | 1.02 | 1.04 | 101.27 |
| | 10.17 | 1.02 | 1.04 | 102.37 |
| | 9.81 | 0.98 | 0.95 | 97.05 |
| | 9.85 | 0.99 | 1.03 | 104.64 |
| | 10.00 | 1.00 | 1.00 | 100.15 |
| | 9.99 | 1.00 | 0.96 | 96.58 |
| | 9.90 | 0.99 | 1.00 | 101.22 |
| | 9.98 | 1.00 | 0.99 | 99.32 |
| | 10.02 | 1.00 | 1.01 | 100.50 |
| **Mean [mg]** | 10.03 | 1.00 | 1.00 | 99.97 |
| Standard deviation [mg] | 0.16 | 0.02 | 0.03 | |
| Relative standard deviation [%] | | | 2.89 | |

Aerodynamic particle size distribution was determined according to Example 1.

Mouthpiece, induction port, preseparator and all stages were individually sampled for active ingredient. A defined volume of solvent was used for each sample.

| | |
|---|---|
| Novolizer® | 10 mL |
| Induction port | 15 mL |
| Preseparator | 20 mL |
| Stage 1-7 | 5 mL |
| MOC | 5 ml |

Tables 17 and 18 summarize the outcome of the NGI testing of powder composition (III), namely the API mass per stage of each single run including a calculated average (Table 17) and presented in a relative manner (Table 18).

**Table 17: API mass per stage; mass distribution; single shot data; Novolizer® loaded with 0.7 ± 0.1 g of powder composition (III).**

| **Deposition data** | 1 Salbutamol sulfate, µg | 2 Salbutamol sulfate, µg | 3 Salbutamol sulfate, µg | Average, µg |
|---|---|---|---|---|
| Novolizer® | 9.30 | 11.17 | 12.85 | 11.10 |
| Induction Port | 25.60 | 2392 | 20.77 | 2343 |
| Preseparator | 26.93 | 25.97 | 34.75 | 29.22 |
| Stage 1 | 5.14 | 6.41 | 6.89 | 6.14 |
| Stage 2 | 16.09 | \|17.93 | 17.30 | 17.11 |
| Stage 3 | 23.43 | 25.53 | 23.80 | 2425 |
| Stage 4 | 21.15 | 21.86 | 19.66 | 20.89 |
| Stage 5 | 7.70 | 9.15 | 6.56 | 7.80 |
| Stage 6 | 1.84 | 2.10 | 1.87 | 1.94 |
| Stage 7 | 0.00 | 0.68 | 0.87 | 0.52 |
| MOC | 0.00 | 1.06 | 0.00 | 0.35 |
| Flow rate, L/min | 78.3 | 78.3 | 78.3 | 78.3 |

**Table 18: API mass per stage; percentage distribution; single shot data; Novolizer® loaded with 0.7 ± 0.1 g powder composition (III).**

| **Deposition data** | 1 Salbutamol sulfate, % | 2 Salbutamol sulfate, % | 3 Salbutamol sulfate, % | Average, % |
|---|---|---|---|---|
| Novolizer® | 6-78 | 7.66 | 8.84 | 7.76 |
| Induction Port | 18.67 | 16.41 | 14.29 | 16.46 |
| Preseparator | 19.63 | 17.82 | 23.92 | 20.45 |
| Stage 1 | 3.75 | 4.40 | 4.74 | 4.29 |
| Stage 2 | 11.73 | 12.30 | 11.91 | 11.98 |
| Stage 3 | 17.08 | 17.52 | 16.38 | 16.99 |
| Stage 4 | 15.42 | 14.99 | 13.53 | 14.65 |
| Stage 5 | 5.61 | 6.27 | 4.52 | 5.47 |
| Stage 6 | 1.34 | 1.44 | 1.29 | 1.36 |
| Stage 7 | 0.00 | 0.47 | 0.60 | 0.35 |
| MOC | 0.00 | 0.73 | 0.00 | 0.24 |
| Flow rate, L/min | 78.3 | 78.3 | 78.3 | 78.3 |

Determined aerodynamic parameters for powder composition (III) are presented in Table 19.

**Table 19: Aerodynamic parameters, powder composition (III).**

| | Run 1 | Run 2 | Run 3 | **Mean/dose** |
|---|---|---|---|---|
| Total Dose Per Shot [ug] | 137.17 | 145.77 | 145.32 | **142.75** |
| Fine Particle Dose [ug] | 62.85 | 69.95 | 61.83 | **64.88** |
| Fine Particle Fraction [%] | 45.81 | 47.99 | 42.55 | **45.45** |
| MMAD [um] | 2.81 | 2.81 | 2.95 | **2.86** |

Tables 20 and 21 summarize the outcome of the NGI testing of powder composition (IV), namely the API mass per stage of each single run including a calculated average (Table 20) and presented in a relative manner (Table 21).

**Table 20: API mass per stage; mass distribution; single shot data; Novolizer® loaded with 0.7 ± 0.1 g of powder composition (IV).**

| **Deposition data** | 1 Salbutamol sulfate, µg | 2 Salbutamol sulfate, µg | 3 Salbutamol sulfate, µg | Average, µg |
|---|---|---|---|---|
| Novolizer® | 1822 | 26.83 | 24.32 | 23.12 |
| Induction Port | 82.69 | 130.60 | 95.44 | 102.91 |
| Preseparator | 43.46 | 47.46 | 32.39 | 41.10 |
| Stage 1 | 9.16 | 17.65 | 10.91 | 12.57 |
| Stage 2 | 41.25 | 71.49 | 51.30 | 54.68 |
| Stage 3 | 66.75 | 114.05 | 88.43 | 89.75 |
| Stage 4 | 61.35 | 105.67 | 65.50 | 77.51 |
| Stage 5 | 22.50 | 80.87 | 30.95 | 44.78 |
| Stage 6 | 5.52 | 9.63 | 7.97 | 7.71 |
| Stage 7 | 0.00 | 0.00 | 0.00 | 0.00 |
| MOC | 1.35 | 0.00 | 0.00 | 0.45 |
| Flow rate, L/min | 78.3 | 78.3 | 78.3 | 78.3 |

**Table 21: API mass per stage; percentage distribution; single shot data; Novolizer® loaded with 0.7 ± 0.1 g of powder composition (IV).**

| **Deposition data** | 1 Salbutamol sulfate, % | 2 Salbutamol sulfate, % | 3 Salbutamol sulfate, % | Average, % |
|---|---|---|---|---|
| Novo | 5.17 | 4.44 | 5.9 | 5.19 |
| Induction Port | 23.48 | 21.61 | 23.44 | 22.84 |
| Preseparator | 12.34 | 7.85 | 7.95 | 9.38 |
| Stage 1 | 2.60 | 2.92 | 2.68 | 2.73 |
| Stage 2 | 11.71 | 11.83 | 12.60 | 12.05 |
| Stage 3 | 18.95 | 18.87 | 21.72 | 19.85 |
| Stage 4 | 17.42 | 17.49 | 16.08 | 17.00 |
| StageS | 6.39 | 13.38 | 7.60 | 9.12 |
| Stage 6 | 1.57 | 1.59 | 1.96 | 1.71 |
| Stage 7 | 0.00 | 0.00 | 0.00 | 0.00 |
| MOC | 0.38 | 0.00 | 0.00 | 0.13 |
| Flow rate, L/min | 78.3 | 78.3 | 78.3 | 78.3 |

Determined aerodynamic parameters for powder composition (IV) are presented in Table 22.

**Table 22: Aerodynamic parameters, powder composition (IV).**

| | Run 1 | Run 2 | Run 3 | **Mean/dose** |
|---|---|---|---|---|
| Total Dose Per Shot [ug] | 352.26 | 60425 | 407.21 | **454.57** |
| Fine Particle Dose [ug] | 181.12 | 350.84 | 222.40 | **251.46** |
| Fine Particle Fraction [%] | 51.42 | 58.06 | 54.62 | **54.70** |
| MMAD [um] | 2.69 | 2.50 | 2.76 | **2.65** |

Component (i) of the powder composition allows high API load, e.g. 1.5 and 10 wt.-%. Further, powder compositions (III) and (IV) showed a homogeneous distribution of the API. Owing to the very low variations in the shot weights, good dosing capability of powder compositions (III) and (IV) with the Novolizer® can be achieved. Moreover, as high FPF and good API distribution during the NGI testing could be observed, excellent aerodynamic properties and lung deposition of powder compositions (III) and (IV) becomes apparent. The small MMAD ensures optimal API deposition in deeper regions of the lung (alveoli).

### Example 5. 6: Powder compositions (V) and (VI)

Powder composition (V) and (VI), respectively, consist of lactose agglomerates according to component (i) of the invention (prepared according to Example 7 in WO 2016/039698) and 1.5 wt.-% budenoside and 10 wt.-% budenoside, respectively, as API according to component (ii) of the invention. In contrast to powder compositions (I) and (II), a low shear mixing method with a Turbula mixer instead of a high shear mixing method was applied.

### Preparation of powder compositions (V) and (VI)

Powder compositions (V) and (VI) were prepared analogously to powder compositions (III) and (IV), except that budenoside was used as API instead of salbutamol sulfate.

### Blend homogeneity and API recovery

Homogeneity was assessed as described for powder compositions (III) and (IV).

Details regarding the HPLC approach are given below:

| | |
|---|---|
| Analyte: | Budenoside / dissolved in a mixture of 25% purified water and 75% methanol. The pH was adjusted to 3.65 with orthophosphoric acid 85% and 22% acetonitrile. |
| Instrumentation: | Agilent 1100 Series HPLC Value System |
| Mobile phase: | 25 wt.-% purified water and 75 wt.-% methanol |
| Flow: | 1 mL/min |
| Column: | Column LiChrospher® 100 RP-18 (5 µm) (Merck, Germany) |
| Wavelength: | 248 nm |
| Retention time: | ∼3.0 min |
| Calibration: | 0.6-150 µg/mL (external standards and samples were injected twice into the HPLC) |
| Injection Volume: | 100 µL |
| Oven temperature: | 25°C |

HPLC experiments for both powder composition (V) and (VI) resulted in an API recovery of 101.88 ± 4.93% and 97.15 ± 2.10%, respectively over all investigated aliquots, and, thus, confirmed excellent blend homogeneity.

### Scanning electron microscopy

Scanning electron microscopy (SEM) was carried out as described for Examples 3 and 4.

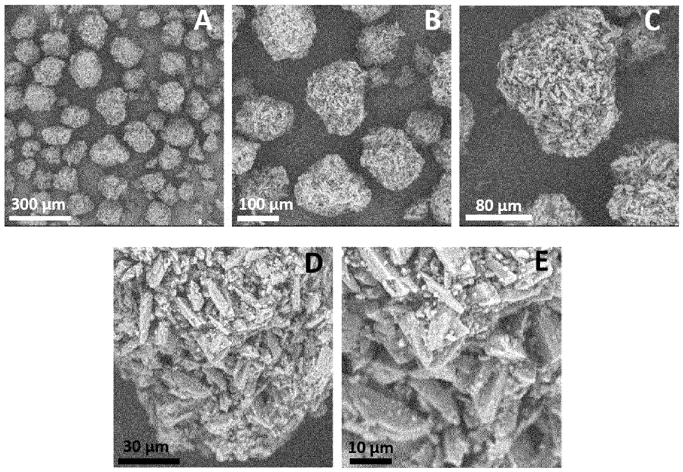

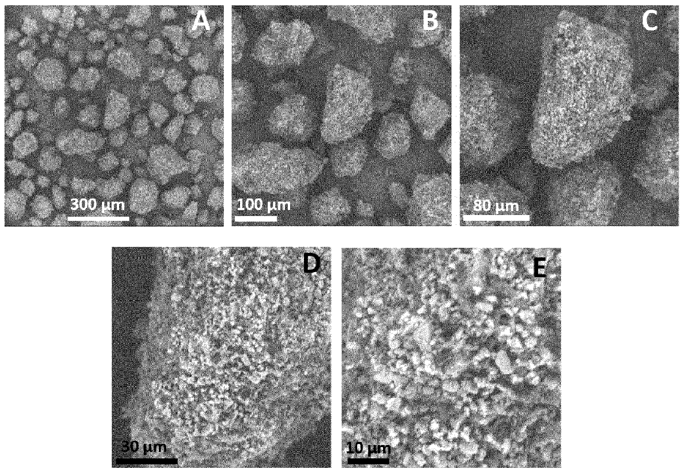

Figure 3 depicts SEM pictures of powder composition (V). Figure 4 depicts SEM pictures of powder composition (VI). For both compositions, spherically agglomerated lactose particles comprising radially arranged prism-like lactose particles carrying API particles thereon become apparent.

### Aerodynamic assessment by NGI

Powder compositions (V) and (VI) were tested for aerodynamic performance analogously to powder compositions (III) and (IV).

### Shot weight test

The amount of API per inhaled dose was determined as presented in Example 1. The following tables list the shot weight each shot number. Reproducible shot weights become apparent for both powder compositions (V) and (VI).

**Table 23: API content and relative standard deviation of 10 random aliquots of powder composition (V).**

| | Sample weight [mg] | Expected API [mg] | Found API [mg] | Recovery [%] |
|---|---|---|---|---|
| | 9.96 | 0.15 | 0.16 | 107.77 |
| | 10.41 | 0.16 | 0.14 | 90.22 |
| | 10.27 | 0.15 | 0.15 | 99.13 |
| | 9.82 | 0.15 | 0.15 | 100.61 |
| | 10.30 | 0.15 | 0.15 | 100.23 |
| | 10.16 | 0.15 | 0.16 | 105.98 |
| | 9.72 | 0.15 | 0.15 | 102.82 |
| | 10.30 | 0.15 | 0.17 | 108.39 |
| | 10.21 | 0.15 | 0.15 | 100.71 |
| | 10.28 | 0.15 | 0.16 | 102.94 |
| | | | | |
| **Mean [mg]** | 10.14 | 0.15 | 0.15 | 101.88 |
| Standard deviation [mg] | 0.23 | 0.00 | 0.01 | |
| Relative standard deviation [%] | | | 4.93 | |

**Table 24: API content and relative standard deviation of 10 random aliquots of powder composition (VI).**

| | Sample weight [mg] | Expected API [mg] | Found API [mg] | Recovery [%] |
|---|---|---|---|---|
| | 10.45 | 1.05 | 1.03 | 98.41 |
| | 10.11 | 1.01 | 1.00 | 98.81 |
| | 10.29 | 1.03 | 0.98 | 95.15 |
| | 10.11 | 1.01 | 0.97 | 96.42 |
| | 10.06 | 1.01 | 0.99 | 98.09 |
| | 10.24 | 1.02 | 1.00 | 97.21 |
| | 10.09 | 1.01 | 0.96 | 95.50 |
| | 9.90 | 0.99 | 0.96 | 97.40 |
| | 10.31 | 1.03 | 0.99 | 95.79 |
| | 9.72 | 0.97 | 0.96 | 98.74 |
| | | | | |
| **Mean [mg]** | 10.13 | 1.01 | 0.98 | 97.15 |
| Standard deviation [mg] | 0.21 | 0.02 | 0.02 | |
| Relative standard deviation [%] | | | 2.10 | |

Tables 25 and 26 summarize the outcome of the NGI testing of powder composition (V), namely the API mass per stage of each single run including a calculated average (Table 25) and presented in a relative manner (Table 26).

**Table 25: API mass per stage; mass distribution; single shot data; Novolizer® loaded with 0.7 ± 0.1 g of powder composition (V).**

| **Deposition data** | 1 Budesonide, µg | 2 Budesonide, µg | 3 Budesonide, µg | Average |
|---|---|---|---|---|
| Novolizer® | 11.17 | 11.39 | 11.18 | 11.25 |
| Induction Port | 21.44 | 20.49 | 18.21 | 20.05 |
| Preseparator | 15.66 | 17.24 | 18.81 | 17.24 |
| Stage 1 | 2.79 | 2.71 | 3.68 | 3.06 |
| Stage 2 | 5.37 | 5.43 | 7.30 | 6.03 |
| Stage 3 | 6.76 | 7.75 | 10.59 | 8.37 |
| Stage 4 | 9.71 | 11.68 | 12.94 | 11.44 |
| Stage 5 | 7.24 | 8.20 | 8.55 | 8.00 |
| Stage 6 | 3.54 | 3.46 | 4.54 | 3.85 |
| Stage 7 | 0.98 | 0.00 | 0.00 | 0.72 |
| MOC | 0.00 | 0.00 | 0.00 | 0.12 |
| Flow rate, L/min | 78.3 | 78.3 | 78.3 | 78.3 |

**Table 26: API mass per stage; percentage distribution; single shot data; Novolizer® loaded with 0.7 ± 0.1 g powder composition (V).**

| **Deposition data** | 1 Budesonide, % | 2 Budesonide, % | 3 Budesonide, % | Average, % |
|---|---|---|---|---|
| Novolizer® | 13.19 | 12.89 | 11.67 | 12.59 |
| Induction Port | 25.33 | 23.19 | 19.00 | 22.51 |
| Preseparator | 18.49 | 19.51 | 19.63 | 19.21 |
| Stage 1 | 3.29 | 3.06 | 3.85 | 3.40 |
| Stage 2 | 6.34 | 6.15 | 7.62 | 6.70 |
| Stage 3 | 7.99 | 8.77 | 11.06 | 9.27 |
| Stage 4 | 11-47 | 13.22 | 13.51 | 12.73 |
| Stage 5 | 8.56 | 9.29 | 8.93 | 8.92 |
| Stage 6 | 4.18 | 3.92 | 4.74 | 4.28 |
| Stage 7 | 1.15 | 0.00 | 0.00 | 0.38 |
| MOC | 0.00 | 0.00 | 0.00 | 0.00 |
| Flow rate, L/min | 78.3 | 78.3 | 78.3 | 78.3 |

Determined aerodynamic parameters for powder composition (V) are presented in Table 27.

**Table 27: Aerodynamic parameters, powder composition (V).**

| | Run 1 | Run 2 | Run 3 | **Mean/dose** |
|---|---|---|---|---|
| Total Dose Per Shot [ug] | 84.66 | 88.35 | 95.82 | **89.61** |
| Fine Particle Dose [ug] | 31.02 | 33.94 | 40.43 | **35.13** |
| Fine Particle Fraction [%] | 36.64 | 38.41 | 42.19 | **39.08** |
| MMAD [um] | 2.08 | 2.10 | 2.27 | **2.15** |

Tables 28 and 29 summarize the outcome of the NGI testing of powder composition (VI), namely the API mass per stage of each single run including a calculated average (Table 28) and in a relative manner (Table 29).

**Table 28: API mass per stage; mass distribution; single shot data; Novolizer® loaded with 0.7 ± 0.1 g powder composition (VI).**

| **Deposition data** | 1 Budesonide, µg | 2 Budesonide, µg | 3 Budesonide, µg | Average, µg |
|---|---|---|---|---|
| Novolizer® | 40.72 | 53.14 | 56.48 | 50.11 |
| Induction Port | 69.26 | 104.22 | 108.42 | 93.97 |
| Preseparator | 69.45 | 113.20 | 120.54 | 101.07 |
| Stage 1 | 8.24 | 12.13 | 14.85 | 11.74 |
| Stage 2 | 14.91 | 22.04 | 25.73 | 20.89 |
| Stage 3 | 27.20 | 37.12 | 42.79 | 35.71 |
| Stage 4 | 47.39 | 61.15 | 71.96 | 60.16 |
| Stage 5 | 37.02 | 46.77 | 52.36 | 45.38 |
| Stage 6 | 17.49 | 21.92 | 20.80 | 20.07 |
| Stage 7 | 3.58 | 6.58 | 3.62 | 4.59 |
| MOC | 0.00 | 1.32 | 0.00 | 0.44 |
| Flow rate, L/min | 78.3 | 78.3 | 78.3 | 78.3 |

**Table 29: API mass per stage; percentage distribution; single shot data; Novolizer® loaded with 0.7 ± 0.1 g of powder composition (VI).**

| **Deposition data** | 1 Budesonide, % | 2 Budesonide, % | 3 Budesonide, % | Average, % |
|---|---|---|---|---|
| Novolizer® | 12.14 | 11.08 | 10.91 | 11.38 |
| Induction Port | 20.66 | 21.73 | 20.95 | 21.11 |
| Preseparator | 20.72 | 23.60 | 23.29 | 22.54 |
| Stage 1 | 2.46 | 2.53 | 2.87 | 2.62 |
| Stage 2 | 4.45 | 4.60 | 4.97 | 4.67 |
| Stage 3 | 8.11 | 7.74 | 8.27 | 8.04 |
| Stage 4 | 14.13 | 12.75 | 13.90 | 13.60 |
| Stage 5 | 11.04 | 9.75 | 10.12 | 10.30 |
| Stage 6 | 5.22 | 4.57 | 4.02 | 4.60 |
| Stage 7 | 1.07 | 1.37 | 0.70 | 1.05 |
| MOC | 0.00 | 0.27 | 0.00 | 0.D9 |
| Flow rate, L/min | 78.3 | 78.3 | 78.3 | 78.3 |

Determined aerodynamic parameters for powder composition (VI) are presented in Table 30.

**Table 30: Aerodynamic parameters, powder composition (VI).**

| | Run 1 | Run 2 | Run 3 | **Mean/dose** |
|---|---|---|---|---|
| Total Dose Per Shot [ug] | 335.26 | 479.59 | 517.55 | **444.13** |
| Fine Particle Dose [ug] | 140.48 | 186.37 | 204.87 | **177.24** |
| Fine Particle Fraction [%] | 41.90 | 38.86 | 39.58 | **40.12** |
| MMAD [um] | 1.82 | 1.86 | 1.95 | **1.88** |

Component (i) of the powder composition allows high API load, e.g. 1.5 and 10 wt.-%. Further, powder compositions (V) and (VI) showed a homogeneous distribution of the API. Owing to the very low variations in the shot weights, good dosing capability of powder compositions (V) and (VI) with the Novolizer® can be achieved. Moreover, as high FPF and good API distribution during the NGI testing could be observed, excellent aerodynamic properties and lung deposition of powder compositions (V) and (VI) becomes apparent. The small MMAD ensures optimal API deposition in deeper regions of the lung (alveoli).

### Example 7: Powder composition (VII)

Powder composition (VII) consists of lactose agglomerates according to component (i) of the invention (prepared according to Example 7 in WO 2016/039698) and 80 wt.-% rifampicin as API according to component (ii) of the invention. In contrast to powder compositions (I) and (II), a low shear mixing method with the Turbula mixer instead of a high shear mixing method was applied.

### Preparation of powder composition (VII)

Rifampicin was pre-sieved with a 180 µm sieve and component (i) with a 355 µm sieve. Afterwards, 4.0 g rifampicin were added to 1.0 g component (i) followed by low shear mixing using the Turbula-Blender (model T2C, Willy Bachofen AG Maschinenfabrik, Basel, Switzerland) for 10 minutes at 90 U/min. After sieving the product with a 355 µm sieve, powder composition (VII) was received.

### Blend homogeneity and API recovery

Blend homogeneity of the powder composition (VII) was determined according to the above Examples.

Homogeneity results are depicted in Table 32. Since only low variations in the total dose per shot were measured, blend homogeneity is apparent.

### Scanning electron microscopy

Scanning electron microscopy (SEM) was carried out as described for Examples 3 and 4.

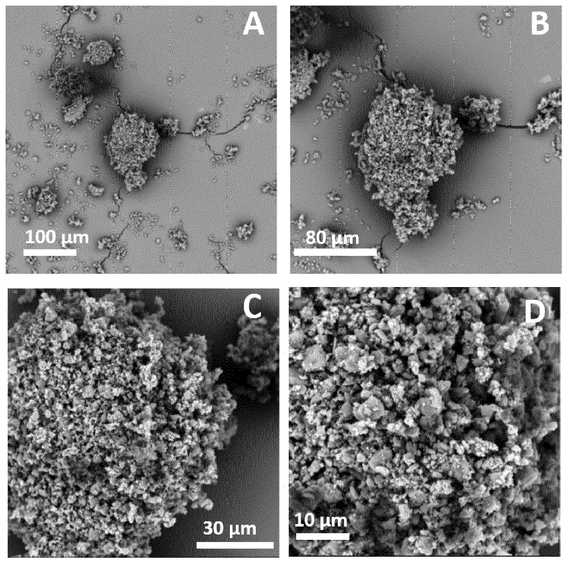

Figure 5 depicts SEM pictures of powder composition (VII). Spherically agglomerated lactose particles comprising radially arranged prism-like lactose particles carrying API particles thereon become apparent.

### Aerodynamic assessment by NGI

Powder composition (VII) was tested for aerodynamic performance with a RS01 inhalation system. This inhalation system is a capsule based system obtained from Plateapem.

Aerodynamic particle size distribution was determined similar to Example 1. The RS01 was loaded with one capsule size 3 filled with 20 mg of powder composition (VII).

Capsule, inhalation system (RS 01), mouthpiece, induction port, preseparator and all stages were individually sampled for active ingredient. A defined volume of solvent was used for each sample.

| | |
|---|---|
| Capsule | 30 mL |
| RS01 | 50 mL |
| Mouthpiece | 10 mL |
| Induction port | 15 mL |
| Preseparator | 30 mL |
| Stage 1-7 | 10 mL |
| MOC | 10 mL |

Tables 31 summarizes the outcome of the NGI testing of powder composition (VII), namely the API mass per stage of each single run including a calculated average.

**Table 31: API mass per stage; mass distribution; single shot data; RS01 loaded with one capsule size 3 filled with 20 mg of powder composition (VII).**

| **Deposition data** | 1 Rifampicin, µg | 2 Rifampicin, µg | 3 Rifampicin, µg | Average |
|---|---|---|---|---|
| Mouthpiece | 101.32 | 121.50 | 128.20 | 117.01 |
| Ind. Port | 1876.75 | 1776.19 | 2056.10 | 1903.01 |
| Preseparator | 623.52 | 627.91 | 574.13 | 608.52 |
| Stage 1 | 1003.67 | 991.70 | 1205.16 | 1066.84 |
| Stage 2 | 2113.00 | 2343.37 | 2787.65 | 2414.67 |
| Stage 3 | 2241.67 | 2342.34 | 2501.40 | 2361.81 |
| Stage 4 | 2228.59 | 2404.46 | 2353.25 | 2328.77 |
| Stage 5 | 1337.31 | 1433.49 | 1385.35 | 1385.38 |
| Stage 6 | 577.64 | 618.60 | 586.69 | 594.31 |
| Stage 7 | 204.28 | 230.34 | 207.79 | 214.14 |
| MOC | 95.66 | 96.61 | 63.25 | 85.17 |
| Flow rate, L/min | 100 | 100 | 100 | 100 |

Determined aerodynamic parameters for powder composition (VII) are presented in Tables 32.

**Table 32: Aerodynamic parameters, powder composition (VII).**

| | Run 1 | Run 2 | Run 3 | Mean/dose |
|---|---|---|---|---|
| Total Dose Per Shot [ug] | 13269.76 | 13954.68 | 14694.99 | 1397.14 |
| Calc Delivered Dose [ug] | 12302.09 | 12865.01 | 13720.77 | 12962.59 |
| Calc Delivered Dose [%] | 92.71 | 92.19 | 93.37 | 92.76 |
| Fine Particle Dose [ug] | 7299.25 | 7103.30 | 6583.75 | 6995.43 |
| Fine Particle Fraction [%] | 66.89 | 68.81 | 66.59 | 67.43 |
| MMAD [um] | 2.39 | 2.37 | 2.59 | 2.45 |
| Recovery [%] | 82.94 | 86.78 | 91.03 | 86.91 |

Component (i) of the powder composition allows high API load, e.g. 80 wt.-%. Further, powder compositions (VII) showed a homogeneous distribution of the API. Moreover, as high FPF and good API distribution during the NGI testing could be observed, excellent aerodynamic properties and lung deposition of powder compositions (VII) become apparent. The small MMAD ensures optimal API deposition in deeper regions of the lung (alveoli).

### Example 8: Comparison of component (i) and commercially available lactose carriers

Component (i) (prepared according to Example 7 in WO 2016/039698) was compared to commercially available lactose carriers INH70, INH120, INH230 and INH250 (Meggle; Wasserburg).

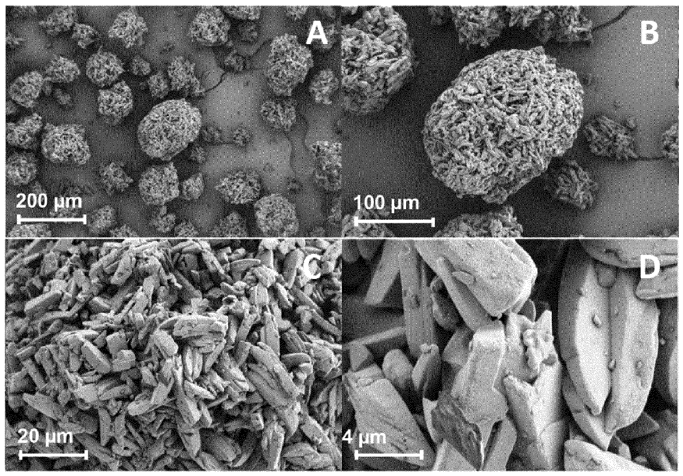

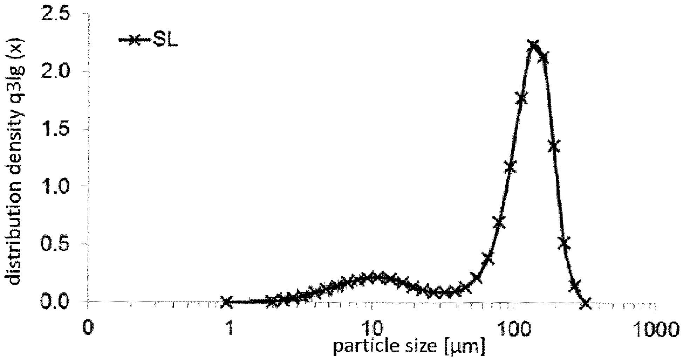

Figure 6 shows SEM pictures of component (i). Spherically agglomerated lactose particles comprising radially arranged prism-like lactose particles are apparent. Further, particle size distribution of component (i) measured by laser diffraction is depicted in Figure 7. d₁₀, dso and d₉₀ values are summarized in Table 33 together with corresponding values of the commercially available lactose carriers.

**Table 34: d₁₀, dso and d₉₀ values, bulk and tapped density of component (i) as well as commercially available lactose carriers.**

| **lactose sample** | **d₁₀ [µm]** | **d₅₀ [µm]** | **d₉₀ [µm]** | **bulk density [g/L]** | **tapped density [g/L]** |
|---|---|---|---|---|---|
| component (i) | 8 | 118 | 198 | n. d. | n. d. |
| INH70 | 135 | 215 | 301 | 600 | 710 |
| INH120 | 88 | 132 | 175 | 720 | 830 |
| INH230 | 45 | 97 | 144 | 700 | 850 |
| INH250 | 13 | 49 | 91 | 640 | 880 |
| INH400 | 1.2 | 7.7 | 27.9 | 0.33 | 0.53 |

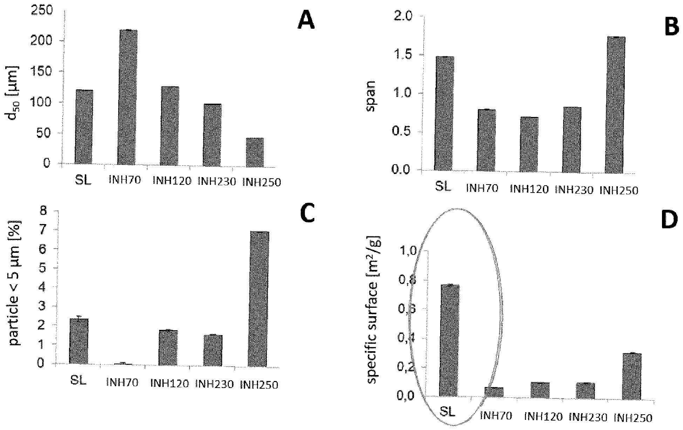

Component (i) exhibits a specific surface of approximately 0.8 m²/g (Figure 8D), thereby exceeding values of all investigated commercially available lactose carriers. Thus, powder compositions of the invention comprising component (i) allow for high enrichment of APIs on the lactose carrier component (i).

### Example 9: Comparison of a powder composition according to the invention and commercially available systems

A powder composition according to the invention consisting of component (i) of Example 8 (prepared according to Example 7 in WO 2016/039698) and 1.5 wt.-% salbutamol sulfate (powder composition (VIII)) was compared to powder compositions consisting of the commercially available lactose carriers INH120 and INH230 of Example 8 and 1.5 wt.-% salbutamol sulfate (comparative powder compositions A and B).

Additionally, the lactose carriers INH120 and INH230 have been mixed with 7.5 wt.-% INH400 (Meggle, Wasserburg; see above) to provide ternary mixtures of powder compositions with salbutamol sulfate for DPls (comparative powder compositions C and D).

The composition of comparative powder compositions is listed in Table 34:

**Table 34: Composition of comparative powder compositions.**

| **comparative powder composition NO.** | **INH120 [wt.-%]** | **INH230 [wt.-%]** | **INH400 [wt.-%]** | **salbutamol sulfate [wt.-%]** |
|---|---|---|---|---|
| A | 98.5 | / | / | 1.5 |
| B | / | 98.5 | / | 1.5 |
| C | 91 | / | 7.5 | 1.5 |
| D | / | 91 | 7.5 | 1.5 |

### Preparation of powder composition (VIII) and comparative powder compositions A-D

Above powder compositions were prepared as described above for powder composition (I).

### Aerodynamic assessment by NGI

Powder composition (VIII) and comparative powder compositions A-D were tested for aerodynamic properties by NGI. Testing was performed similar to the above-presented NGI approach. As inhaler system, a Novolizer® system was used.

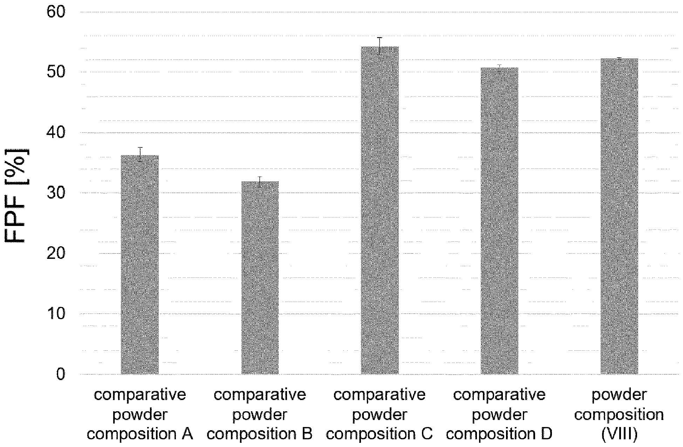

As presented in Figure 9, powder composition (VIII) reached FPF values higher than values measured for comparative powder compositions A and B and similar to values measured for comparative powder compositions C and D (ternary mixtures).

Consequently, powder compositions according to the invention reach similar FPF values than commercially available ternary powder compositions or even exceed these values. Advantageously, powder compositions according to the invention - contrary to the comparisons C and D - do not require the additional step of preparing ternary mixtures.

The present invention relates to the following embodiments:
1. A powder composition comprising:
   (i) spherically agglomerated lactose particles comprising radially arranged prism-like lactose particles,
   (ii) at least one active pharmaceutical ingredient (API), and
   (iii) optionally at least one additive.
2. The powder composition according to item 1, wherein component (i) comprises α-lactose, β-lactose or combinations thereof, preferably α-lactose.
3. The powder composition according to item 1 or 2, wherein component (i) comprises anhydrous lactose, lactose monohydrate or combinations thereof, preferably lactose monohydrate.
4. The powder composition according to any of the preceding items, wherein component (i) is obtained according to a process comprising the steps:
   (a) preparing a lactose solution,
   (b) adding the lactose solution obtained after step (a) to at least one non-solvent under constant stirring to form spherically agglomerated lactose particles comprising radially arranged prism-like lactose particles,
   (c) isolating the spherically agglomerated lactose particles, and
   (d) optionally drying.
5. The powder composition according to claim 4, wherein the lactose solution in step (a) is an aqueous lactose solution.
6. The powder composition according to claim 4 or 5, wherein the non-solvent in step (b) is selected from the group consisting of methanol, ethanol, n-propanol, 2-propanol and n-butanol, preferably 2-propanol and ethanol, more preferably ethanol.
7. The powder composition according to any of the preceding items, wherein component (i) has an average particle size from 50-1,000 µm, preferably from 100-400 µm.
8. The powder composition according to any of the preceding items, wherein component (i) has a dso value from 50-250 µm, preferably from 75-200 µm.
9. The powder composition according to any of the preceding items, wherein component (i) has a specific surface area from 0.5-5.0 m²/g, preferably from 0.6-3.5 m²/g.
10. The powder composition according to any of the preceding items, wherein the API in component (ii) is selected from a respiratory and a systemic active pharmaceutical ingredient, preferably for the treatment of diseases of the respiratory system, diseases caused by bacteria, diseases of the cardiovascular system, allergic reactions, cancer, psychiatric diseases and immune system related diseases.
11. The powder composition according to item 10, wherein the active pharmaceutical ingredient for the treatment of diseases of the respiratory system is selected from the group consisting of inhaled corticosteorides (ICS), short acting beta agonists (SABA), long acting beta agonists (LABA), short acting anticholinergic (SAMA), long acting anticholinergic (LAMA), bronchodilators and antitussives, preferably salbutamol, budenoside, formoterol, salmeterol, ipatropiumbromid, terbutaline, tiotropium, indaceterol, umcledinium, glycopyrronium, aclidinium, beclometasone, fluticasone, vilanterol and pharmaceutically acceptable salts thereof.
12. The powder composition according to item 10, wherein the active pharmaceutical ingredient for the treatment of diseases caused by bacteria is selected from the group consisting of β-lactams, fluorquinolones, macrolides, tetracyclines, aminoglycosides, lincosamides, oxazolidinones, and glycopeptides, preferably rifampicin, amoxiclin, moxifloxacin, levofloxacin, clarithromycin, doxycyclin, amipicillin, cefuroxim, ceftriaxon, cefotaxim, etrapenem, metropenem, imipenem, ciprofloxacin, gentamicin, tobraycin, amikacin, ceftobiprol, ceftazidium, flucloaxacillin, penicillin, cefazolin, clindamycin, linezolid, vacomiycin and pharmaceutically acceptable salts thereof.
13. The powder composition according to item 10, wherein the active pharmaceutical ingredient for the treatment of diseases of the cardiovascular system is selected from the group consisting of ACE inhibitors, aldosterone receptor antagonists, beta blockers, cardiac glycosides, diuretics, neprilysin inhibitors and organic nitro compounds, preferably benazapril, captopril, cilazapril, enalapril, ramipril, spironolactone, canrenone, eplerenone, propranolol, bucindolol, acebutolol, bisoprolol, butaxamine, nebivolol, amiloride, triamterene, nitroglycerine and pharmaceutically acceptable salts thereof.
14. The powder composition according to item 10, wherein the active pharmaceutical ingredient for the treatment of allergic reactions is selected from the group consisting of antihistamines and mast cell stabilizers, preferably acrivastine, astemizole, cetirizine, ebastine, loratadine, mizolastine, terfenadine, rupatadine, cromoglicic acid, nedocromil and pharmaceutically acceptable salts thereof.
15. The powder composition according to item 10, wherein the active pharmaceutical ingredient for the treatment of cancer is selected from the group consisting of alkylating antineoplastic agents, platinum analogs, anthracyclines, mitosis inhibitors, taxanes, topoisomerase inhibitors, kinase inhibitors, cellular-based ingredients, mRNA-based ingredients, DNA-based ingredients, vector-based ingredients, polyplex-based ingredients, protein-based ingredients, monoclonal antibodies and polyclonal antibodies, preferably cyclophosphamide, carboplatin, cisplatin, idarubicin, daunorubicin, doxorubicin, mitoxantron, epirubicin, pixantron, paclitaxel, docetaxel, cabazitaxel and pharmaceutically acceptable salts thereof.
16. The powder composition according to item 10, wherein the active pharmaceutical ingredient for the treatment of psychiatric diseases is selected from the group consisting of antidepressants, mood stabilizers, neuroleptics and anxiolytics, preferably agomelatine, paroxetine, citalopram, amitriptyline, aripiprazole, clozapine, olanzapine, quetiapine, risperidone and pharmaceutically acceptable salts thereof.
17. The powder composition according to item 10, wherein the active pharmaceutical ingredient for the treatment of immune system related diseases is an immune suppressive API, preferably selected from the group consisting of glucocorticoid, cytostatic agent, antibody and API acting on immunophilins.
18. The powder composition according to any of the preceding items, wherein component (ii) has a particle size distribution dso of 1-10 µm, preferably 1-5 µm.
19. The powder composition according to any of the preceding items, wherein the API of component (ii) is adsorbed on component (i).
20. The powder composition according to any of the preceding items, wherein the additive of component (iii) is selected from a lubricant and a stabilizing agent.
21. The powder composition according to item 20, wherein the lubricant is magnesium stearate.
22. The powder composition according to item 20 or 21, wherein the stabilizing agent is a carbohydrate, amino acid or metal salt, preferably lactose, mannitol, trehalose or leucine.
23. The powder composition according to any of the preceding items, wherein the overall powder composition has a dso value in the range from 50-1000 µm, preferably from 50-300 µm, more preferably from 50-150 µm.
24. The powder composition according to any of the preceding items, wherein the overall powder composition has a fine particle fraction (FPF) ranging from 20-90%, preferably from 30-80%, more preferably from 40-70%.
25. The powder composition according to any of the preceding items, wherein the overall powder composition has a mass median aerodynamic diameter (MMAD) ranging from 0.5-5 µm, preferably from 1-4 µm, more preferably from 1.5-3 µm.
26. The powder composition according to any of the preceding items, wherein the amount of component (i) in the powder composition is in the range of 50-99.9 wt.-%, preferably in the range of 80-99.9 wt.-%, based on the total amount of components (i) and (ii).
27. The powder composition according to any of the preceding items, wherein the amount of component (ii) in the powder composition is in the range from 0.1-50 wt.-%, preferably in the range from 0.1-20 wt.-%, based on the total amount of components (i) and (ii).
28. The powder composition according to any of the preceding items, wherein component (iii) in the powder composition is present in the range from 0.1-20 wt.-%, preferably in the range from 0.1-10 wt.-%, based on the total amount of components (i), (ii) and (iii).
29. The powder composition according to any of the preceding items, wherein the powder composition is not in form of a tablet or injective, preferably not in form of a tablet.
30.A method of preparing a powder composition according to items 1-29, comprising the steps of:
   (1) adding components (ii) and optionally (iii) to component (i),
   (2) mixing the product obtained after step (1), and
   (3) optionally sieving the mixture obtained after step (2).
31. The method according to item 30, wherein step (2) is performed in a stirrer, high shear mixer, low shear mixer and/or by co-processing.
32. The method according to item 30 or 31, wherein a sieve having a mesh size in the range from 30-500 µm, preferably from 150-400 µm is used in step (3).
33. The powder composition according to any of the items 1-29 for use as inhalable medicament and/or inhalable diagnostic agent.
34. The powder composition according to to any of the items 1-29 and 33 for use in the treatment of diseases of the respiratory system, diseases caused by bacteria, diseases of the cardiovascular system, allergic reactions, cancer, psychiatric diseases and/or immune system related diseases.
35. A use of component (i) as carrier in inhaler compositions.

## Claims

1. A powder composition comprising:
(i) spherically agglomerated lactose particles comprising radially arranged prism-like lactose particles,
(ii) at least one active pharmaceutical ingredient (API), and
(iii) optionally at least one additive.

2. The powder composition according to claim 1, wherein component (i) comprises α-lactose, β-lactose or combinations thereof, preferably α-lactose, and/or
wherein component (i) comprises anhydrous lactose, lactose monohydrate or combinations thereof, preferably lactose monohydrate.

3. The powder composition according to claim 1 or 2, wherein component (i) is obtained according to the process comprising the steps:
(a) preparing a lactose solution,
(b) adding the lactose solution obtained after step (a) to at least one non-solvent under constant stirring to form spherically agglomerated lactose particles comprising radially arranged prism-like lactose particles,
(c) isolating the spherically agglomerated lactose particles, and
(d) optionally drying.

4. The powder composition according to any of the preceding claims, wherein component (i) has an average particle size from 50-1,000 µm, preferably from 100-400 µm, and/or
wherein component (i) has a dso value from 50-250 µm, preferably from 75-200 µm, and/or
wherein component (i) has a specific surface area from 0.5-5.0 m²/g, preferably from 0.6-3.5 m²/g.

5. The powder composition according to any of the preceding claims, wherein the API in component (ii) is selected from a respiratory and a systemic active pharmaceutical ingredient, preferably for the treatment of diseases of the respiratory system, diseases caused by bacteria, diseases of the cardiovascular system, allergic reactions, cancer, psychiatric diseases and immune system related diseases.

6. The powder composition according to any of the preceding claims, wherein component (ii) has a particle size distribution dso of 1-10 µm, preferably 1-5 µm.

7. The powder composition according to any of the preceding claims, wherein the API of component (ii) is adsorbed on component (i).

8. The powder composition according to any of the preceding claims, wherein the overall powder composition has a dso value in the range from 50-1000 µm, preferably from 50-300 µm, more preferably from 50-150 µm, and/or
wherein the overall powder composition has a fine particle fraction (FPF) ranging from 20-90%, preferably from 30-80%, more preferably from 40-70%, and/or
wherein the overall powder composition has a mass median aerodynamic diameter (MMAD) ranging from 0.5-5 µm, preferably from 1-4 µm, more preferably from 1.5-3 µm.

9. The powder composition according to any of the preceding claims, wherein the amount of component (i) in the powder composition is in the range of 50-99.9 wt.-%, preferably in the range of 80-99.9 wt.-%, based on the total amount of components (i) and (ii).

10. The powder composition according to any of the preceding claims, wherein the amount of component (ii) in the powder composition is in in the range from 0.1-50 wt.-%, preferably in the range from 0.1-20 wt.-%, based on the total amount of components (i) and (ii).

11. The powder composition according to any of the preceding claims, wherein component (iii) in the powder composition is present in the range from 0.1-20 wt.-%, preferably in the range from 0.1-10 wt.-%, based on the total amount of components (i), (ii) and (iii).

12. A method of preparing a powder composition according to claims 1-11, comprising the steps of:
(1) adding components (ii) and optionally (iii) to component (i),
(2) mixing the product obtained after step (1), and
(3) optionally sieving the mixture obtained after step (2).

13. The powder composition according to any of the claims 1-11 for use as inhalable medicament and/or inhalable diagnostic agent.

14. The powder composition according to any of the claims 1-11 and 13 for use in the treatment of diseases of the respiratory system, diseases caused by bacteria, diseases of the cardiovascular system, allergic reactions, cancer, psychiatric diseases and/or immune system related diseases.

15. A use of component (i) as carrier in inhaler compositions.
